# EUROPEAN PATENT SPECIFICATION

(11) **EP 0 877 740 B1**
(45) Date of publication and mention of the grant of the patent: **30.03.2005**
(21) Application number: 96944583.2
(22) Date of filing: 17.12.1996
(51) Int. Cl.: C07D 277/30, A61K 31/425, C07D 213/55, C07D 307/54, C07D 239/26, C07D 333/24, C07D 241/12, C07D 417/12

(54) **THIOL DERIVATIVES WITH METALLOPEPTIDASE INHIBITORY ACTIVITY**
THIOL-DERIVATIVE MIT METALLOPEPTIDASE HEMMENDER WIRKUNG
DERIVES THIOL PRESENTANT UNE ACTIVITE INHIBITRICE DE METALLOPEPTIDASE

(30) Priority: 28.12.1995 IT MI952773
(43) Date of publication of application: 18.11.1998
(73) Proprietor: ZAMBON GROUP S.p.A., 36100 Vicenza (IT)
(72) Inventor: PELLACINI, Franco, I-20151 Milano (IT); FANTUCCI, Mario, I-20144 Milano (IT); NORCINI, Gabriele, I-21010 Vizzola Ticino (IT); ROMAGNANO, Stefano, I-20090 Buccinasco (IT); SANTANGELO, Francesco, I-20148 Milano (IT); SEMERARO, Claudio, I-20091 Bresso (IT)
(74) Representative: Longoni, Alessandra
(86) International application number: PCT/EP1996/005663
(87) International publication number: WO 1997/024342

(56) References cited:
- EP-A- 0 419 327
- EP-A- 0 539 848
- WO-A-96/22998
- US-A- 4 401 677
- J.MED.CHEM., vol. 37, 1994, pages 1070-1083, XP002029219 FOURNI -ZALUSKI: "New dual inhibitors of neutral ..."
- BIOORG. MED. CHEM. LETT., vol. 5, no. 7, 1995, pages 735-738, XP002029220 BHAGWHAT ET AL.: "Alpha-Mercaptoacyl dipeptides that inhibit angiotensin ..." cited in the application
- BIOORG. MED. CHEM. LETT. (BMCLE8,0960894X);96; VOL.6 (19); PP.2317-2322, HOECHST MARION ROUSSEL;CENTRE RECHERCHES ROUSSEL UCLAF; ROMAINVILLE; 93235; FR. (FR), XP002029221 DEPREZ P ET AL: "Thiol inhibitors of endothelin-converting enzyme"

## Description

The present invention relates to thiol derivatives with metallopeptidase inhibitory activity and, more particularly, it relates to N-mercaptoacyl phenylalanine derivatives useful in the treatment of cardiovascular diseases.

The pharmacologic interest towards the study of metallopeptidase inhibitory molecules derives from the role that said enzymes exert on the level of the cardiocirculatory system.

It is well-known in fact that compounds with angiotensin converting enzyme (ACE) inhibitory activity are mainly useful in the treatment of hypertension, heart failure and post-infarct in that they inhibit the formation of angiotensin II, a substance which produces several effects among which the increase of the blood pressure.

Compounds with endothelin converting enzyme (ECE) inhibitory activity are useful as anti-vasoconstrictors because they inhibit the formation of endothelin, a 21 amino acid peptide with vasoconstrictor activity.

Instead, compounds with inhibitory activity of the neutral endopeptidase (NEP) enzyme, also called enkephalinase, are useful as vasodilators since the NEP enzyme is responsible for the inactivation, not only of endogenous enkephaline, but also of some natriuretic factors such as, for instance, the atrial factor (ANF), a hormone secreted by heart which increases the vasodilatation and, on the renal level, increases diuresis and natriuresis.

Therefore, even exerting their action on the cardiovascular system with different mechanisms of action, the compounds with metallopeptidase inhibitory activity are generally used, alone or in combination, in the treatment of hypertension, renal failure, congestive heart failure and ischemic cardiopathologies.

Among the thiol derivatives inhibitors of metallopeptidases, Thiorphan [(DL-(3-mercapto-2-benzylpropanoyl)glycine], described for the first time by Roques et al. in Nature, Vol. 288, pages 286-288, (1980), and Captopril (The Merck Index, XI ed., No. 1773, page 267) are considered the parent compounds for NEP-inhibitors and ACE-inhibitors, respectively.

Other molecules having thiol structure endowed with metallopeptidase inhibitory activity are described in the literature.

The US patents No. 4,401,677 and No. 4,199,512 (both in the name of E.R. Squibb & Sons, Inc.) describe mercaptoalkanoyl amino acids endowed with enkephalinase inhibitory activity and ACE-inhibitory activity, respectively.

The European patent application No. 0419327 (Société Civile Bioproject) describes amino acid derivatives endowed with enkephalinase and ACE inhibitory activity, respectively.

The European patent application No. 0449523 (E.R Squibb & Sons, Inc.) describes mercapto or acylthio trifluoromethylamides with NEP-inhibitory activity.

The international patent application No. WO 93/08162 [Rhone-Poulenc Rorer S.A. - Institut National de la Santé et de la Recherche Médicale (INSERM)] describes β,β-disubstituted α-mercaptomethylpropionylamides endowed with mixed ACE/NEP inhibitory activity.

The European patent application No. 0524553 [Institut National de la Santé et de la Recherche Médicale (INSERM)] describes acylmercaptoalkanoyldipeptides endowed with neutral endopeptidase and peptidyldipeptidase A inhibitory activity.

The European patent application No. 0566157 (Schering Corporation) describes thiol derivatives such as, for instance, N-mercaptoacyl amino acids, endowed with NEP-inhibitory activity.

α-Mercaptoacyl dipeptides endowed with ACE-inhibitory and NEP-inhibitory activity are also described by S.S. Bhagwat et al. in Bioorganic & Medicinal Chemistry Letters, 7, 735-738, 1995.

In this last work the authors conclude that, while the presence of a biphenylmethyl group confers an interesting mixed ACE/NEP-inhibitory activity at the molecules having an α-mercaptoacyl dipeptide structure, the substitution of the biphenyl group with groups such as α- or β-naphthyl causes a significant loss of activity.

J. Med. Chem. (1994) 37, pages 1070-1083, reports a study assisted by molecular modelling carried out with the aim of finding dual ACE/NEP inhibitors. The work did not make into consideration the influence of the substitutents on the phenyl moiety in the C-terminal amino acid. EP-A-0539848 describes certain ACE/NEP inhibitors which encompass also some substituents on the phenyl ring in the C-terminal amino acid but the examples relate only to 4-OH substitution, i.e. to the natural amino acid tyrosine.

Now we have found N-mercaptoacyl phenylalanine derivatives which are endowed with a remarkable inhibitory activity on the angiotensin converting enzyme as well as on the neutral endopeptidase enzyme (mixed or dual ACE/NEP inhibitory activity) which makes them particularly useful in the therapy of cardiovascular pathologies.

Therefore, object of the present invention are the compounds of formula 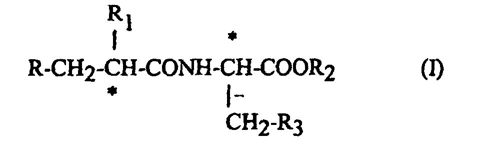 wherein
- R: is a mercapto group or a R₄COS group convertible in the organism to mercapto group;
- R₁: is a straight or branched C₂-C₄ alkyl group or an aryl or arylalkyl group having from 1 to 6 carbon atoms in the alkyl moiety wherein the aryl is a phenyl or a 5 or 6 membered aromatic heterocycle with one or two heteroatoms selected from the group consisting of nitrogen, oxygen and sulphur, optionally substituted with one or more substituents, the same or different, selected from the group consisting of halogen atoms, hydroxy groups, alkoxy, alkyl, alkylthio, alkylsulphonyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, wherein the acyl group is that of an alyphatic or aromatic carboxylic acid selected from acetic, propionic, butyric and benzoic acid, aminosulphonyl groups, mono- or di-alkylamino or mono- or di-alkylaminocarbonyl groups having from 1 to 6 carbon atoms in the alkyl moiety;
- R₂: is a hydrogen atom, a straight or branched C₁-C₄ alkyl or a benzyl group;
- R₃: is a phenyl group substituted with a 5 or 6 membered aromatic heterocycle with one or two heteroatoms selected from the group consisting of nitrogen, oxygen and sulphur, being the phenyl and the heterocyclic groups optionally substituted with one or more substituents, the same or different, as indicated for R₁;
- R₄: is a straight or branched C₁-C₄ alkyl group or a phenyl group;
the carbon atoms marked with an asterisk are stereogenic centers;
and pharmaceutically acceptable salts thereof.

The compounds of formula I contain two stereogenic centers and can thus exist in the form of stereoisomers.

Therefore, object of the present invention are the compounds of formula I in the form of stereoisomeric mixture as well as in the form of single stereoisomers.

The compounds of formula I object of the present invention are endowed with a mixed ACE/NEP-inhibitory activity and are useful in the treatment of cardiovascular diseases.

Although both terms mixed and dual are indifferently used, the term dual is the one more commonly accepted in the literature for compounds endowed contemporaneously with ACE-and NEP-inhibitory activity.

In the present context, the terms mixed and dual are to be considered equivalent.

In the present description, unless otherwise specified, with the term straight or branched alkyl group we intend an alkyl such as methyl, ethyl, n.propyl, isopropyl, n.butyl, see-butyl. tert-butyl, isobutyl, n.pentyl, 2-pentyl, 3-pentyl, isopentyl, tert-pentyl, n.hexyl and isohexyl group; with the term straight or branched alkoxy group we intend an alkoxy such as methoxy, ethoxy, n.propoxy and isopropoxy group; with the term halogen atom we intend a fluorine, chlorine, bromine or iodine atom; with the term acyl we intend an acyl group deriving from an aliphatic or aromatic carboxylic acid such as acetic, propionic, butyric and benzoic acid; with the term 5 or 6 membered aromatic heterocycle containing 1 or 2 heteroatoms selected among nitrogen, oxygen and sulphur we intend a group such as thiazole, isoxazole, oxazole, isothiazole, pyrazole, imidazole, thiophene, pyrrole, pyridine, pyrimidine, pyrazine, pyridazine and furan, optionally benzocondensed.

Examples of pharmaceutically acceptable salts of the compounds of formula I are the salts with alkali or alkali-earth metals and the salts with pharmaceutically acceptable organic bases.

Preferred compounds of formula I are the compounds wherein R₃ is a phenyl group substituted in position 4 with a heterocyclic group.

More preferred compounds, in this class, are the compounds of formula I wherein R₁ represents an arylalkyl group optionally substituted with one or more substituents, the same or different, selected from the group consisting of halogen atoms, hydroxy, alkyl or alkoxy.

Still more preferred compounds, in this class, are the compound of formula I wherein R₁ represents a phenylalkyl group optionally substituted with one or more substitutents, the same or different, selected from the group consisting of halogen atoms, hydroxy, alkyl or alkoxy.

Preferred examples of pharmaceutically acceptable salts of the compounds of formula I are the salts with alkali metals such as sodium, lithium and potassium.

It is clear to the man skilled in the art that the compounds of formula I wherein R is a R₄COS group convertible in the organism to mercapto group, as well as the compounds of formula I wherein R₂ is an alkyl or benzyl group, are biologic precursors (pro-drugs) of the corresponding compounds of formula I wherein R is a mercapto group (R=SH) or R₂ is a hydrogen atom (R₂=H), respectively.

Specific examples of preferred compounds of formula I, object of the present invention, are:
N-(3-phenylcarbonylthio-2-phenylmethylpropionyl)-4-(2-thiazolyl)-phenylalanine methyl ester;
N-(3-phenylcarbonylthio-2-phenylmethylpropionyl)-4-(2-pyridyl)-phenylalanine methyl ester;
N-(3-phenylcarbonylthio-2-phenylmethylpropionyl)-4-(3-pyridyl)-phenylalanine benzyl ester;
N-(3-phenylcarbonylthio-2-phenylmethylpropionyl)-4-(2-furyl)-phenylalanine methyl ester;
N-(3-phenylcarbonylthio-2-phenylmethylpropionyl)-4-(5-pyrimidinyl)phenylalanine ethyl ester;
N-(3-phenylcarbonylthio-2-phenylmethylpropionyl)-4-(2-pyrazinyl)-phenylalanine methyl ester;
N-(3-phenylcarbonylthio-2-phenylmethylpropionyl)-4-(2-thienyl)-phenylalanine methyl ester;
N-(3-phenylcarbonylthio-2-phenylmethylpropionyl)-4-(3-thienyl)-phenylalanine methyl ester;
N-(3-phenylcarbonylthio-2-phenylmethylpropionyl)-4-(3-furyl)-phenylalanine methyl ester;
N-[3-phenylcarbonylthio-2-(3-pyridylmethyl)propionyl]-4-(2-thiazolyl)-phenylalanine methyl ester;
N-[3-acetylthio-2-(3-methoxyphenyl)methyl-propionyl]-4-(2-thiazolyl)-phenylalanine methyl ester;
N-[3-acetylthio-2-(2-fluorophenyl)methyl-propionyl]-4-(2-thiazolyl)-phenylalanine methyl ester;
N-[3-phenylcarbonylthio-2-(2-thienyl)methyl-propionyl]-4-(2-thiazolyl)-phenylalanine methyl ester;
N-[2-(2-furyl)methyl-3-phenylcarbonylthio-propionyl]-4-(2-thiazolyl)-phenylalanine methyl ester;
N-[2-(3-methyl-5-isoxazolyl)methyl-3-phenylcarbonylthio-propionyl]-4-(2-thiazolyl)-phenylalanine methyl ester;
N-(3-mercapto-2-phenylmethylpropionyl)-4-(2-thiazolyl)-phenylalanine;
N-(3-mercapto-2-phenylmethylpropionyl)-4-(2-pyridyl)-phenylalanine;
N-(3-mercapto-2-phenylmethylpropionyl)-4-(3-pyridyl)-phenylalanine;
N-(3-mercapto-2-phenylmethylpropionyl)-4-(2-furyl)-phenylalanine;
N-(3-mercapto-2-phenylmethylpropionyl)-4-(5-pyrimidinyl)-phenylalanine;
N-(3-mercapto-2-phenylmethylpropionyl)-4-(2-pyrazinyl)-phenylalanine;
N-(3-mercapto-2-phenylmethylpropionyl)-4-(2-thienyl)-phenylalanine;
N-(3-mercapto-2-phenylmethylpropionyl)-4-(3-thienyl)-phenylalanine;
N-(3-mercapto-2-phenylmethylpropionyl)-4-(3-furyl)-phenylalanine;
N-[3-mercapto-2-(3-pyridylmethyl)propionyl]-4-(2-thiazolyl)-phenylalanine;
N-[3-mercapto-2-(3-methoxyphenyl)methyl-propionyl]-4-(2-thiazolyl)-phenylalanine;
N-[3-mercapto-2-(2-thienyl)methyl-propionyl]-4-(2-thiazolyl)-phenylalanine;
N-[3-mercapto-2-(3-methyl-5-isoxazolyl)methyl-propionyl]-4-(2-thiazolyl)-phenylalanine;
N-[2-(2-fluorophenyl)methyl-3-mercapto-propionyl]-4-(2-thiazolyl)-phenylalanine;
N-[2-(2-furyl)methyl-3-mercapto-propionyl]-4-(2-thiazolyl)-phenylalanine.

The preparation of the compounds of formula I, object of the present invention, is carried out according to a synthetic process comprising the reaction between a compound of formula 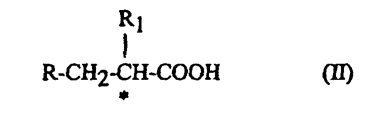 wherein
R and R₁ have the above reported meanings;
and a phenylalanine derivative of formula 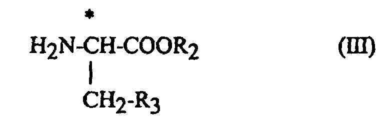 wherein
R₂ and R₃ have the above reported meanings.

The condensation reaction is carried out according to conventional techniques of the chemistry of peptides.

Before carrying out the reaction it can be useful to properly protect the optional functional groups which could interfere in the reaction.

The optional protection is carried out according to conventional techniques.

In this respect the compounds wherein R is a R₄COS group are preferably used as intermediates of formula II, thus obtaining the corresponding compounds of formula I wherein R=R₄COS from which, by hydrolysis, the compounds of formula I wherein R=SH can be obtained.

The evaluation of the usefulness of the optional protection as well as the selection of the kind of adopted protection, according to the reaction to be carried out and to the functional groups to be protected, are within the normal knowledge of the man skilled in the art.

The removal of the optional protective groups is carried out according to conventional techniques.

For a general reference to the use of protective groups in organic chemistry see Theodora W.

Greene and Peter G.M. Wuts "Protective Groups in Organic Synthesis", John Wiley & Sons, Inc., II Ed., 1991.

The compounds of formula II are known or easily prepared according to conventional methods as described, for instance, in British patent No. 1576161 in the name of E.R. Squibb & Sons Inc.

Alternatively, the compounds of formula II can be prepared according to the synthetic methods described by M.C. Fournié-Zalusky et al. in J. Med. Chem. **1994**, 37, 1070-1083.

Also the intermediate compounds of formula III are known or easily prepared according to known methods.

For instance, the compounds of formula III can be prepared according to the synthetic methods described by W. C. Shieh et al. in J. Org. Chem. 1992, 57, 379-381.

Alternatively, the compounds of formula III can be prepared by coupling methods (cross-coupling) starting from halogenated heterocyclic compounds and stannyl phenylalanine derivatives, as described by D.S. Wilbur et al. in Bioconjugate Chem., 1993, 4, 574-580.

The compounds of formula I in the form of single stereoisomers are prepared by stereoselective synthesis or by separation of the stereoisomeric mixture according to conventional techniques.

Also the preparation of the salts of the compounds of formula I, object of the present invention, is carried out according to conventional techniques.

The compounds of formula I, object of the present invention, are endowed with a mixed ACE/NEP-inhibitory activity and are useful in the treatment of cardiovascular diseases.

The inhibitory activity of the compounds of formula I was evaluated by means of in vitro tests (example 8).

In particular, the inhibitory activity of the compounds of formula I was evaluated in comparison to the aforementioned Thiorphan and Captopril.

The in vitro inhibitory activity of the compounds of formula I, expressed as IC₅₀ value, resulted at nM concentrations.

Said activity resulted to be comparable to that of Captopril, to what it concerns the ACE-inhibitory activity, and greater than that of Thiorphan, to what it concerns the NEP-inhibitory activity.

The inhibitory activity of the compounds of formula I, moreover, was also evaluated by means of ex-vivo tests in comparison to known compounds (example 9).

In particular, N-[3-mercapto-2-(3,4-methylenedioxyphenyl)methyl-propionyl]-(S)-phenylalanine and N-[3-mercapto-2-(3,4-methylenedioxyphenyl)methyl-propionyl)-(S)-tyrosine, described as enkephalinase and ACE-inhibitors in the aforementioned European patent application No. 0419327 and hereinafter referred to as compounds R-1 and R-2 respectively, as well as N-(3-mercapto-2-methylpropanoyl)-L-tyrosine and N-(3-mercapto-2-methylpropanoyl)-L-triptophan, described as ACE-inhibitors in U.S. 4,199,512 and as enkephalinase inhibitors in U.S. 4,401,677 and hereinafter referred to as compounds R-3 and R-4 respectively, were used as comparison compounds.

The ex-vivo ACE/NEP-inhibitory activity, in particular, was determined by evaluating the enzymatic activity in tissue homogenates (lung and kidney for ACE and NEP activity, respectively) from spontaneously hypertensive rats (SHR) treated with the tested compounds by intravenous or oral route.

It is worth noting that the activity shown by the compounds of formula I in the ex vivo tests confirms the mixed activity (dual activity) shown in the in vitro tests, also after oral administration.

Said activity, moreover, resulted to be significantly higher than that of the comparison compounds.

For a practical use in therapy, the compounds of formula I can be formulated in solid or liquid pharmaceutical compositions, suitable to oral or parenteral administration.

Therefore, the pharmaceutical compositions containing a therapeutically effective amount of a compound of formula I in admixture with a carrier for pharmaceutical use are a further object of the present invention.

Specific examples of pharmaceutical compositions according to the present invention are tablets, coated tablets, capsules, granulates, solutions and suspensions suitable to oral administration, solutions and suspensions suitable to parenteral administration.

The pharmaceutical compositions object of the present invention are prepared according to conventional techniques.

The daily dose of the compound of formula I or of the corresponding pro-drug will depend on several factors such as the seriousness of the disease, the individual response of the patient or the kind of formulation but it is usually comprised between 0.1 mg and 10 mg per kg of body weight divided into a single dose or into more daily doses.

With the aim of illustrating the present invention the following examples are now given.

Unless otherwise specified, the flash chromatographies were carried out by using flash chromatography silica gel from Baker company (code 7024-00).

### Example 1

### Preparation of N-tert-butoxycarbonyl-4-(5-pyrimidinyl)-L-phenylalanine ethyl ester

A mixture of 5-pyrimidinylboronic acid (850 mg; 2 mmoles), N-tert-butoxycarbonyl-4-trifluoromethylsulphonyl-L-phenylalanine ethyl ester (450 mg; 2.2 mmoles), a solution of sodium carbonate (530 mg) in water (2.59 ml) and a mixture of toluene:ethanol=10:4.5 (20 ml) was degassed with nitrogen for 30 minutes.

Subsequently, palladium(0)tetrakis(triphenylphosphine) (120 mg; 0.06 mmoles) was therein added and the reaction mixture was heated at 90°C and kept under stirring for 3 hours.

The mixture was then kept at room temperature and N-tert-butoxycarbonyl-4-trifluoromethylsulphonyl-L-phenylalanine ethyl ester (112 mg) and palladium(0)tetrakis-(triphenylphosphine) (30 mg) were added again.

The mixture was further heated at the temperature of 90°C and kept under stirring for other 18 hours.

After cooling the reaction mixture at room temperature, ethyl acetate (100 ml) and water (40 ml) were added.

The phases were separated and the organic phase was dried on sodium sulphate and evaporated under vacuum. The obtained residue was purified by flash chromatography (silica gel, eluent hexane:ethyl acetate=7:3, pressure of nitrogen 0.1 atm) thus affording N-tert-butoxycarbonyl-4-(5-pyrimidinyl)-L-phenylalanine ethyl ester (130 mg; 14% yield) as a colourless oil.
¹H-NMR (200 MHz, CDCl₃): δ (ppm): 1.24 (t, 3H, CH₂-CH₃); 1.40 [s, 9H, C(CH₃)₃): 3.01-3.25 (m, 2H, CH₂-CH); 4.19 (q, 2H, CH₂-CH₃); 4.52-4.65 (m, 1H, CH-COO); 5.06 (d, 1H, NH); 7.25-7.52 (m, 4H, phenylene); 8.91 (s, 2H, N-CH-C-CH-N); 9.19 (s, 1H, N-CH-N).

### Example 2

### Preparation of N-tert-butoxycarbonyl-4-(2-thiazolyl)-L-phenylalanine methyl ester

N-tert-butoxycarbonyl-4-(trifluoromethylsulphonyl)-L-phenylalanine methyl ester (8 g; 32.3 mmoles) and palladium-bis(triphenylphosphine) chloride (2.3 g) were added to a solution of 2-trimethylstannyl-thiazole (13.8 g; 32.3 mmoles) in a mixture of tetrahydrofuran: toluene= 1 0: 1 (50 ml), previously degassed with nitrogen.

The mixture was refluxed for 24 hours and, subsequently, 2-trimethylstannyl-thiazole (2 g) was added again.

After 6 hours at reflux N-tert-butoxycarbonyl-4-(trifluoromethylsulphonyl)-L-phenylalanine methyl ester (2 g) and palladium-bis(triphenylphosphine) chloride (700 mg) were added.

The resultant reaction mixture was kept under stirring at 70°C for 16 hours and, subsequently, cooled at room temperature.

Water (200 ml) was then added to the mixture which was extracted with methylene chloride (4x200 ml).

The collected organic phases were dried on sodium sulphate and evaporated under vacuum.

The obtained residue was purified by flash chromatography (silica gel, eluent methylene chloride:ethyl acetate=9:1, pressure of nitrogen=0.1 atm) thus affording N-tert-butoxycarbonyl-4-(2-thiazolyl)-L-phenylalanine methyl ester (2.3 g; 20% yield).
¹H-NMR (200 MHz, CDCl₃): δ (ppm): 1.40 [s, 9H, C(CH₃)₃]; 3.00-3.21 (m, 2H, CH₂); 3.70 (s, 3H, COOCH₃); 4.42-4.65 (m, 1H, CH-COO); 5.02 (bd, 1H, NH); 7.30 (d, 1H, S-CH-CH-N); 7.81 (d, 1H, S-CH-CH-N); 7.15-7.90 (m, 4H, phenylene).

### Example 3

### Preparation of N-tert-butoxycarbonyl-4-(3-pyridyl)-L-phenylalanine methyl ester

3-Bromopyridine (1.67 g; 10 mmoles) and palladium(0)tetrakis(triphenylphosphine) (370 mg; 0.219 mmoles) were added to a solution of N-tert-butoxycarbonyl-4-(tributylstannyl)-L-phenylalanine methyl ester (4 g; 7.03 mmoles), prepared as described by D.S. Wilbur et al. in Bioconjugate Chem., 1973, 4, 574-580, in anhydrous dimethylformamide (30 ml), previously degassed with nitrogen.

The reaction mixture was kept under stirring for 10 minutes at room temperature and, subsequently, heated at 105°C for 6 hours.

Palladium(0)tetrakis(triphenylphosphine) (0.0035 mmoles) was again added and the mixture was kept under stirring at 105°C for 8 hours and then cooled at room temperature.

Water (100 ml) was therein added and the reaction mixture was extracted with hexane (3x 150 ml).

The collected organic phases were washed with a saturated aqueous solution of potassium fluoride, dried on sodium sulphate and evaporated under vacuum.

The obtained residue was collected with ethyl acetate and filtered.

The resultant solution was evaporated under vacuum and the residue was purified by flash chromatography (silica gel, eluent hexane:ethyl acetate=8:2, pressure of nitrogen 0.1 atm) affording N-tert-butoxycarbonyl-4-(3-pyridyl)-L-phenylalanine methyl ester (1.5 g; 60% yield) as a colourless oil.
Mass (C.I.): (M+H)⁺=357
¹H-NMR (200 MHz, CDCl₃): δ (ppm): 1.40 [s, 9H, C(CH₃)₃]; 3.00-3.20 (m, 2H, CH₂); 3.71 (s, 3H, COOCH₃); 4.55 (m, 1H, CH-COO); 5.05 (bd, 1H, NH); 7.19-7.51 (m, 4H, phenylene); 7.30-8.82 (bm, 4H, pyridyl).

By working in an analogous way the following compounds were prepared:

### N-tert-butoxycarbonyl-4-(2-pyridyl)-L-phenylalanine methyl ester

Mass (C.I.): (M+H)⁺=357
¹H-NMR (200 MHz, CDCl₃): δ (ppm): 1.40 [s, 9H, C(CH₃)₃]; 3.03-3.21 (m, 2H, CH₂-CH); 3.70 (s, 3H, COOCH₃); 4.54-4.65 (m, 1H, CH-COO); 4.98 (d, 1H, CONH); 7.16-7.21 (m, 1H, N-C-CH-CH); 7.65-7.77 (m, 2H, N-CH-CH-CH); 7.19-7.93 (m, 4H, phenylene); 8.63-8.68 (m, 1H, N-CH);

### N-tert-butoxycarbonyl-4-(2-pyrazinyl)-L-phenylalanine methyl ester

¹H-NMR (200 MHz, CDCl₃): δ (ppm): 1.40 [s, 9H, C(CH₃)₃]; 3.02 (m, 2H, CH₂); 3.70 (s, 3H, COOCH₃); 4.53-4.70 (m, 1H, CH-COO); 5.03 (bd, 1H, NH); 7.21-7.98 (m, 4H, phenylene); 8.49 and 8.62 [2(bs, 2H, N-CH-CH-N)]; 9.00 (s, 1H, CH-N-CH-CH);

### N-tert-butoxycarbonyl-4-(2-thienyl)-L-phenylalanine methyl ester

¹H-NMR (200 MHz, CDCl₃): δ (ppm): 1.41 [m, 9H, C(CH₃)₃]; 2.98-3.18 (m, 2H, CH₂); 3.71 (s, 3H, COOCH₃); 4.53-4.64 (m, 1H, CH-COO); 4.98 (bd, 1H, NH); 7.02-7.28 (m, 3H, thienyl); 7.10-7.54 (m, 4H, phenylene).

### Example 4

### Preparation of 4-(3-pyridyl)-L-phenylalanine methyl ester dihydrochloride

Thionyl chloride (0.85 ml; 4.78 mmoles) was added dropwise to a solution of N-tert-butoxycarbonyl-4-(3-pyridyl)-L-phenylalanine methyl ester (1.4 g; 3.93 mmoles) in methanol (30 ml), prepared as described in example 3, keeping the temperature at 0°C.

At the end of the addition, the reaction mixture was brought at room temperature and kept under stirring for 8 hours.

The solvent was then evaporated under vacuum affording 4-(3-pyridyl)-L-phenylalanine methyl ester dihydrochloride (820 mg; 71% yield) used as such in the following reactions.
Mass (C.I.):(M+H)⁺= 257 (free base)
¹H-NMR (200 MHz, D₂O): δ (ppm): 3.11-3.32 (m, 2H, CH₂); 3.67 (s, 3H, CH₃); 4.31-4.37 (m, 1H, CH); 7.30-7.63 (m, 4H, phenylene); 7.97 (dd, 1H, CH-N-CH-CH-CH); 8.59 (d, 1H, CH-N-CH-CH-CH); 8.65-8.71 (m, 1H, CH-N-CH-CH); 8.89 (d, 1H, CH-N-CH-CH-CH).

By working in an analogous way the following compounds were prepared:

### 4-(2-pyridyl)-L-phenylalanine methyl ester dihydrochloride

Mass (C.I.): (M+H)⁺= 257 (free base)
¹H-NMR (200 MHz, D₂O): δ (ppm): 3.12-3.33 (m, 2H, CH₂); 3.64 (s, 3H, CH₃); 4.30-4.37 (m, 1H, CH); 7.36-7.73 (m, 4H, phenylene); 7.78-8.58 (m, 4H, pyridyl);

### 4-(5-pyrimidinyl)-L-phenylalanine ethyl ester dihydrochloride

¹H-NMR (200 MHz, DMSO-d₆): δ (ppm): 1.11 (t, 3H, CH₂-CH₃); 3.10-3.35 (m, 2H, CH₂-CH); 4.04-4.20 (m, 2H, CH₂-CH₃); 4.22-4.35 (m, 1H, CH); 7.40-7.84 (m, 4H, phenylene); 9.15 (s, 2H, N-CH-C-CH-N); 9.19 (s, 1H, N-CH-N);

### 4-(2-pyrazinyl)-L-phenylalanine methyl ester dihydrochloride

Mass (C.I.): (M+H)⁺= 258 (free base)
¹H-NMR (200 MHz, DCl 1N): δ (ppm): 3.45-3.67 (m, 2H, CH₂); 3.98 (s, 3H, CH₃); 4.65-4.72 (m, 1H, CH); 7.68-8.26 (m, 4H, phenylene); 9.02 (d, 1H, CH-N-CH-CH); 9.44 (dd, 1H, CH-N-CH-CH); 9.54 (d, 1H, CH-N-CH-CH);

### 4-(2-thienyl)-L-phenylalanine methyl ester hydrochloride

Mass (C.I.): (M+H)⁺= 262 (free base)
¹H-NMR (200 MHz, D₂O): δ (ppm): 2.98-3.19 (m, 2H, CH₂); 3.65 (s, 3H, CH₃); 4.21-4.28 (m, 1H, CH); 6.96-7.28 (m, 3H, thienyl); 7.08-7.52 (m, 4H, phenylene);

### 4-(2-thiazolyl)-L-phenylalanine methyl ester dihydrochloride

¹H-NMR (200 MHz, D₂O): δ (ppm): 3.10-3.32 (m, 2H, CH₂-CH); 3.68 (s, 3H, CH₃); 4.30-4.38 (m, 1H, CH); 7.30-7.80 (m, 4H, phenylene); 7.70-7.91 (m, 2H, thiazolyl).

### Example 5

### Preparation of 2-isobutyl-3-phenylcarbonylthio-propionic acid

A mixture of 2-isobutyl-acrylic acid (6.34 g; 49 mmoles) and thiobenzoic acid (5.96 ml; 51 mmoles) was heated at 100°C under stirring for 2 hours.

The reaction mixture was then treated with petroleum ether 40-60°C (100 ml) and cooled at -70°C in a dry ice/acetone bath.

By filtration and washing with petroleum ether at -70°C a residue was collected which, dried at reduced pressure, furnished 2-isobutyl-3-phenylcarbonylthio-propionic acid (11.12 g; 85% yield) as a white solide.
¹H-NMR (200 MHz, CDCl₃): δ (ppm): 0.90-1.00 (m, 6H); 1.40-1.90 (m, 3H); 2.70-2.90 (m, 1H); 3.10-3.40 (m, 2H); 7.35-7.62 (m, 3H); 7.90-8.00 (d, 2H).

By working in an analogous way the following compounds were prepared:

### 2-(3-methoxyphenyl)methyl-3-phenylcarbonylthio-propionic acid

¹H-NMR (200 MHz, CDCl₃): δ (ppm): 2.32 (s, 3H); 2.80-3.15 (m, 5H); 3.77 (s, 3H); 6.70-6.80 (m, 3H); 7.14-7.25 (m, 1H).

### 3-acetylthio-2-(2-fluorophenyl)methyl-propionic acid

¹H-NMR (200 MHz, CDCl₃): δ (ppm): 2.31 (s, 3H); 2.90-3.20 (m, 5H); 6.95-7.30 (m, 4H). 3-phenylcarbonylthio-2-(2-thienyl)methyl-propionic acid benzylamine salt
¹H-NMR (200 MHz, DMSO-d₆): δ (ppm): 2.45-3.25 (m, 5H, S-CH₂-CH-CH₂); 3.90 (s, 2H, CH₂-phenyl); 6.85-7.91 (m, 13H, aryl).

### Example 6

### Preparation of N-[(2S)-3-phenylcarbonylthio-2-phenylmethylpropionyl]-4-(2-thiazolyl)-L-phenylalanine methyl ester (compound 1)

A solution of hydroxybenzotriazole (0.54 g; 4 mmoles) in tetrahydrofuran (30 ml) and, subsequently, a solution of dicyclohexylcarbodiimide (0.825 g; 4 mmoles) in methylene chloride (15 ml) were added, at 0°C under stirring, to a mixture of (2S)-3-phenylcarbonyithio-2-phenylmethylpropionic acid (1.2 g; 4 mmoles), 4-(2-thiazolyl)-L-phenylalanine methyl ester dihydrochloride (1.34 g; 4 mmoles), prepared as described in example 4, triethylamine (1.11 ml; 8 mmoles) in tetrahydrofuran (20 ml) and methylene chloride (30 ml).

The reaction mixture was kept under stirring for 20 hours, then dicyclohexyl urea was filtered off and the solvent was evaporated at reduced pressure.

The residue was collected with ethyl acetate and the solution was washed with an aqueous solution of sodium chloride at 20%, sodium bicarbonate at 5% and sodium chloride at 20% again.

After separation of the phases and evaporation of the organic phase, the resultant white solid was purified by flash chromatography (silica gel, eluent ethyl acetate:hexane=40:60, pressure of nitrogen 0.1 atm) thus affording N-[(2S)-3-phenylcarbonylthio-2-phenylmethylpropionyl]-4-(2-thiazolyl)-L-phenylalanine methyl ester (1.5 g).
m.p. 98-100°C
Mass (C.I.): (M+H)⁺= 545
¹H-NMR (200 MHz, CDCl₃): δ (ppm): 2.63-3.35 (m, 7H, CH₂-CH-CH₂, CH₂-C₆H₄-thiazolyl); 3.68 (s, 3H, COOCH₃); 4.75-4.85 (m, 1H, CH-COO); 5.78 (d, 1H, NH); 7.10-8.00 (m, 16H, aryl).

By working in an analogous way, starting from compounds known or prepared as described in examples 4 and 5, the following compounds were obtained:

### N-[(2S)-3-phenylcarbonylthio-2-phenylmethylpropionyl]-4-(2-furyl)-L-phenylalanine methyl ester (compound 2)

m.p. 132-134°C
Mass (C.I.) (M+H)⁺= 528
¹H-NMR (200 MHz, CDCl₃): δ (ppm): 2.60-3.35 (m, 7H, CH₂-CH-CH₂, CH₂-C₆H₄-furyl); 3.58 (s, 3H, COOCH₃); 4.71-4.81 (m, 1H, CH-COO); 5.73 (d, 1H, NH); 6.40-8.00 (m, 17H, aryl);

### N-[(2S)-3-phenylcarbonylthio-2-phenylmethylpropionyl]-4-(5-pyrimidinyl)-L-phenylalanine ethyl ester (compound 3)

m.p. 117-119°C
Mass (C.I.) (M+H)⁺= 554
¹H-NMR (200 MHz, CDCl₃): δ (ppm): 1.18 (t, 3H, CH₃-CH₂); 2.65-3.35 (m, 7H, CH₂-CH-CH₂, CH₂-C₆H₄-pyrimidinyl); 3.95-4.20 (m, 2H, COOCH₂); 4.70-4.80 (m, 1H, CH-COO); 5.78 (d, 1H, NH); 7.05-8.00 (m, 14H, phenyl, phenylene); 8.68 (s, 2H, CH-N-CH-N-CH); 9.11 (s, 1H, N-CH-N);

### N-[(2S)-3-phenylcarbonylthio-2-phenylmethylpropionyl]-4-(2-pyrazinyl)-L-phenylalanine methyl ester (compound 4)

m.p. 145-147°C
Mass (C.I.) (M+H)⁺= 540
¹H-NMR (200 MHz, CDCl₃): δ (ppm): 2.65-3.35 (m, 7H, CH₂-CH-CH₂, CH₂-C₆H₄-pyrazinyl); 3.61 (s, 3H, COOCH₃); 4.75-4.85 (m, 1H, CH-COO); 5.78 (d, 1H, NH); 7.10-8.00 (m, 14H, phenyl, phenylene); 8.48-8.75 (m, 3H, CH-N-CH-CH-N);

### N-[(2S)-3-phenylcarbonylthio-2-phenylmethylpropionyl]-4-(3-pyridyl)-L-phenylalanine methyl ester (compound 5)

m.p. 132-134°C
Mass (C.I.) (M+H)⁺= 539
¹H-NMR (200 MHz, CDCl₃): δ (ppm): 2.66-2.79 (m, 1H, CH₂-CH-CH₂); 2.88-3.35 (m, 6H, CH₂-CH-CH₂, CH₂-C₆H₄-pyridyl); 3.61 (s, 3H, COOCH₃); 4.75-4.85 (m, 1H, CH-COO); 5.77 (d, 1H, NH); 7.07-7.99 (m, 16H, CH-CH-CH-N, phenyl, phenylene); 8.51-8.64 (m, 2H, CH-N-CH);

### N-[(2S)-3-phenylcarbonylthio-2-phenylmethylpropionyl]-4-(2-pyridyl)-L-phenylalanine methyl ester (compound 6)

m.p. 123-125°C
Mass (C.I.) (M+H)⁺= 539
¹H-NMR (200 MHz, CDCl₃): δ (ppm): 2.63-2.77 (m, 1H, CH₂-CH-CH₂); 2.85-3.35 (m, 6H, CH₂-CH-CH₂, CH₂-C₆H₄-pyridyl); 3.56 (s, 3H, COOCH₃); 4.75-4.85 (m, 1H, CH-COO); 5.75 (d, 1H, NH); 7.09-7.99 (m, 16H, CH-CH-CH-N, phenyl, phenylene); 8.61-8.65 (m, 1H, N-CH-CH);

### N-[(2S)-3-phenylcarbonylthio-2-phenylmethylpropionyl]-4-(2-thienyl)-L-phenylalanine methyl ester (compound 7)

Mass (C.I.) (M+H)⁺= 544
¹H-NMR (200 MHz, CDCl₃): δ (ppm): 2.64-3.36 (m, 7H, CH₂-CH-CH₂, CONH-CH-CH₂); 3.58 (s, 3H, COOCH₃); 4.74-4.83 (m, 1H, CH-COO); 5.74 (d, 1H, NH); 6.97-7.99 (m, 17H, aryl);

### N-[(2S)-3-phenylcarbonylthio-2-phenylmethylpropionyl]-4-(3-thienyl)-L-phenylalanine methyl ester (compound 8)

Mass (C.I.) (M+H)⁺= 544
¹H-NMR (200 MHz, CDCl₃): δ (ppm): 2.63-3.35 (m, 7H, CH₂-CH-CH₂, NH-CH-CH₂); 3.58 (s, 3H, COOCH₃); 4.73-4.85 (m, 1H, CH-COO); 5.70-5.76 (bd, 1H, NH); 7.00-7.62 (m, 15H, phenyl, phenylene, CH-CH-S); 7.93-8.00 (m, 2H, CH-S-CH);

### N-[(2S)-3-phenylcarbonylthio-2-phenylmethylpropionyl]-4-(3-furyl)-L-phenylalanine methyl ester (compound 9)

m.p. 115-117°C
Mass (C.I.) (M+H)⁺= 528
¹H-NMR (200 MHz, CDCl₃): δ (ppm): 2.62-3.36 (m, 7H, CH₂-CH-CH₂, NH-CH-CH₂); 3.58 (s, 3H, COOCH₃); 4.71-4.82 (m, 1H, CH-COO); 5.72 (bd, 1H, NH); 6.50-8.00 (m, 17H, aryl);

### N-[3-phenylcarbonylthio-2-(3-pyridylmethyl)propionyl]-4-(2-thiazolyl)-L-phenylalanine methyl ester - stereoisomer A (compound 10)

Mass (C.I.) (M+H)⁺= 546
TLC (ethyl acetate:petroleum ether=95:5), Rf = 0.33
¹H-NMR (200 MHz, CDCl₃): δ (ppm): 2.60-3.38 (m, 7H, CH₂-CH-CH₂, NH-CH-CH₂); 3.60 (s, 3H, COOCH₃); 4.79-4.90 (m, 1H, CH-COO); 6.21 (bd, 1H, NH); 7.29-7.81 (m, 2H, thiazolyl); 6.75-8.48 (m, 13H, phenyl, phenylene, pyridyl);

### N-[3-phenylcarbonylthio-2-(3-pyridylmethyl)propionyl]-4-(2-thiazolyl)-L-phenylalanine methyl ester - stereoisomer B (compound 11)

Mass (C.I.) (M+H)⁺= 546
TLC (ethyl acetate:petroleum ether=95:5), Rf = 0.24
¹H-NMR (200 MHz, CDCl₃): δ (ppm): 2.61-3.25 (m, 7H, CH₂-CH-CH₂, NH-CH-CH₂); 3.60 (s, 3H, COOCH₃); 4.80-4.91 (m, 1H, CH-COO); 6.09 (bd, 1H, NH); 7.27-7.80 (m, 2H, thiazoly); 7.10-8.40 (m, 13H, phenyl, phenylene, pyridyl);

### N-[2-isobutyl-3-phenylcarbonylthio-propionyl]-4-(2-thiazolyl)-L-phenylalanine methyl ester (compound 12)

¹H-NMR (200 MHz, CDCl₃): δ (ppm): 0.80-0.95 (m, 6H); 1.30-1.80 (m, 3H); 2.40-2.60 (m, 1H); 3.00-3.30 (m, 4H); 3.70 (d, 3H); 4.90-5.05 (m, 1H); 6.00-6.15 (bt, 1H); 7.10-8.00 (m, 11H);

### N-[3-acetylthio-2-(3-methoxyphenyl)methyl-propionyl]-4-(2-thiazolyl)-L-phenylalanine methyl ester (compound 13)

¹H-NMR (200 MHz, CDCl₃): δ (ppm): 2.30 (d, 3H); 2.45-3.20 (m, 7H); 3.63 (d, 3H); 3.75 (d, 3H); 4.70-4.93 (m, 1H); 5.70-5.90 (dd, 1H); 6.60-6.85 (m, 4H); 7.17-7.32 (m, 3H); 7.68-7.90 (m, 3H);

### N-[3-acetylthio-2-(3-fluorophenyl)methyl-propionyl]-4-(2-thiazolyl)-L-phenylalanine methyl ester (compound 14)

¹H-NMR (200 MHz, CDCl₃): δ (ppm): 2.29 (s, 3H); 2.55-3.20 (m, 7H); 3.65 (2s, 3H); 4.70-4.90 (m, 1H); 5.80-6.00 (2d, 1H); 6.70-7.32 (m, 3H);

### N-[3-phenylcarbonylthio-2-(2-thienyl)methyl-propionyl]-4-(2-thiazolyl)-L-phenylalanine methyl ester (compound 15)

¹H-NMR (200 MHz, CDCl₃): δ (ppm): 2.68-3.37 (m, 7H); 3.60-3.61 (2s, 3H, COOCH₃); 4.31-4.45 (m, 1H, CH-COOCH₃); 6.01-6.10 (2d, 1H, NH); 6.80-8.00 (m, 14H);

### Example 7

### Preparation of N-[(2S)-3-mercapto-2-phenylmethylpropionyl]-4-(2-thiazolyl)-L-phenylalanine (compound 16)

N-[(2S)-3-phenylcarbonylthio-2-phenylmethylpropionyl]-4-(2-thiazolyl)-L-phenylalanine methyl ester (1.4 g; 2.57 mmoles), prepared as described in example 6, was suspended in ethanol (30 ml), degassed by nitrogen bubbling to eliminate the oxygen.

An aqueous degassed solution of sodium hydroxide 1N (7.7 ml) and, at the end of the addition, further degassed ethanol (20 ml) were added dropwise at 5°C to the suspension.

The reaction mixture was kept under stirring for 4 hours at room temperature, then cooled at 0°C and acidified with hydrochloric acid 5% (10 ml).

The reaction mixture was evaporated to dryness and the residue was collected with acetonitrile and water and, subsequently, was filtered thus affording N-[(2S)-3-mercapto-2-phenylmethylpropionyl]-4-(2-thiazolyl)-L-phenylalanine (1 g).
m.p. 180-182°C
Mass (C.I.) (M+H)⁺= 427
¹H-NMR (200 MHz, DMSO-d₆): δ (ppm): 1.80-1.88 (m, 1H, SH); 2.22-2.84 (m, 5H, CH₂-CH-CH₂); 2.86-3.18 (m, 2H, CH₂-CH-COO); 4.46-4.57 (m, 1H, CH-COO); 7.10-7.25 (m, 5H, phenyl); 7.32-7.84 (m, 4H, phenylene); 7.74 (d, 1H, N-CH=CH-S); 7.89 (d, 1H, N-CH=CH-S); 8.35 (d, 1H, NH); 12.76 (s, 1H, COOH);

By working in an analogous way the following compounds were prepared:

### N-[(2R)-3-mercapto-2-phenylmethylpropionyl]-4-(2-tiazolyl)-L-phenylalanine (compound 17)

¹H-NMR (200 MHz, DMSO-d₆): δ (ppm): 2.15-2.23 (m, 1H, SH); 2.31-2.74 (m, 5H, CH₂-CH-CH₂); 2.78-3.07 (m, 2H, CH₂-CH-COO); 4.42-4.53 (m, 1H, CH-COO); 7.02-7.80 (m, 9H, phenyl and phenylene); 7.75 (d, 1H, N-CH=CH-S); 7.90 (d, 1H, N-CH=CH-S); 8.36 (d, 1H, NH);

### N-[(2S)-3-mercapto-2-phenylmethylpropionyl]-4-(2-furyl)-L-phenylalanine (compound 18) m.p.153-155°C

Mass (C.I.) (M+H)⁺= 410
¹H-NMR (200 MHz, CDCl₃): δ (ppm): 1.40 (t, 1H, SH); 2.45-3.25 (m, 7H, CH₂-CH-CH₂, CH₂-CH-COO); 4.80-4.90 (m, 1H, CH-COO); 5.86 (d, 1H, NH); 6.42-7.42 (m, 3H, furyl); 7.07-7.57 (m, 9H, phenyl, phenylene);

### N-[(2S)-3-mercapto-2-phenylmethylpropionyl]-4-(5-pyrimidinyl)-L-phenylalanine (compound 19)

m.p. 193-195°C
Mass (C.I.) (M+H)⁺= 422
¹H-NMR (200 MHz, DMSO-d₆): δ (ppm): 1.81 (bm, 1H, SH); 2.21-3.20 (m, 7H, CH₂-CH-CH₂, CH₂-C₆H₄-pyrimidinyl); 4.46-4.57 (m, 1H, CH-COO); 7.06-7.29 (m, 5H, phenyl); 7.36-7.72 (m, 4H, phenylene); 8.33 (d, 1H, NHCO); 9.08 (s, 2H, N-CH-C-CH-N); 9.15 (s, 1H, N-CH-N);

### N-[(2S)-3-mercapto-2-phenylmethylpropionyl]-4-(2-pyrazinyl)-L-phenylalanine (compound 20)

mp. 176-178°C
Mass (C.I.) (M+H)⁺= 422
¹H-NMR (200 MHz, DMSO-d₆): δ (ppm): 1.84 (bt, 1H, SH); 2.21-3.21 (m, 7H, CH₂-CH-CH₂, CH₂-CH-COO); 4.48-4.59 (m, 1H, CH-COO); 7.10-7.26 (m, 5H, phenyl); 7.37-8.05 (m, 4H, phenylene); 8.37 (d, 1H, NHCO); 8.58 (d, 1H, CH-N-CH-CH-N); 8.68-8.70 (m, 1H, CH-N-CH-CH-N); 9.21 (d, 1H, C-CH-N); 12.78 (b, 1H, COOH);

### N-[(2S)-3-mercapto-2-phenylmethylpropionyl]-4-(3-pyridyl)-L-phenylalanine (compound 21)

m.p. 146-148°C
Mass (C.I.) (M+H)⁺= 421
¹H-NMR (200 MHz, DMSO-d₆): δ (ppm): 1.69-1.89 (b, 1H, SH); 2.22-3.18 (m, 7H, CH₂-CH-CH₂, CH₂-phenylene); 4.45-4.56 (m, 1H, CH-COO); 7.09-7.26 (m, 5H, phenyl); 7.42-7.48 (m, 1H, CH-N-CH-CH-CH); 7.62-7.33 (m, 4H, phenylene); 7.98-8.04 (m, 1H, CH-N-CH-CH-CH); 8.30 (d, 1H, CONH); 8.53 (dd, 1H, CH-N-CH-CH-CH); 8.83 (d, 1H, CH-N-CH-CH-CH);

### N-[(2S)-3-mercapto-2-phenylmethylpropionyl]-4-(2-pyridyl)-L-phenylalanine (compound 22)

m.p. 157-159°C
Mass (C.I.) (M+H)⁺= 421
¹H-NMR (200 MHz, DMSO-d₆): δ (ppm): 1.87 (b, 1H, SH); 2.23-3.19 (m, 7H, S-CH₂-CH-CH₂, CONH-CH-CH₂); 4.44-4.45 (m, 1H, CH-COO); 7.09-7.93 (m, 8H, N-CH-CH-CH-CH, phenyl); 7.30-7.99 (m, 4H, phenylene); 8.29 (d, 1H, CONH); 8.61-8.65 (m, 1H, C-N-CH);

### N-[(2S)-3-mercapto-2-phenylmethylpropionyl]-4-(2-thienyl)-L-phenylalanine (compound 23)

m.p. 152-154°C
¹H-NMR (200 MHz, CDCl₃): δ (ppm): 1.34-1.43 (m, 1H, SH); 2.44-3.26 (m, 7H, S-CH₂-CH-CH₂, CONH-CH-CH₂); 4.80-4.89 (m, 1H, NH-CH-COO); 5.81 (d, 1H, NH); 7.02-7.52 (m, 12H, aryl);

### N-[(2S)-3-mercapto-2-phenylmethylpropionyl]-4-(3-thienyl)-L-phenylalanine (compound 24)

m.p. 169-171°C
Mass (C.I.) (M+H)⁺= 426
TLC (ethyl acetate:hexane:acetic acid=50:50:5), Rf= 0.44
¹H-NMR (200 MHz, CDCl₃): δ (ppm): 1.84 (bs, 1H, SH); 2.23-3.13 (m, 7H, S-CH₂-CH-CH₂, NH-CH-CH₂); 4.42-4.53 (m, 1H, NH-CH-COO); 7.12-7.80 (m, 12H, aryl); 8.30 (d, 1H, JHH=8.2 Hz, NH);

### N-[(2S)-3-mercapto-2-phenylmethylpropionyl]-4-(3-furyl)-L-phenylalanine (compound 25) m.p. 140-142°C

Mass (C.I.) (M+H)⁺= 410
TLC (ethyl acetate:hexane:acetic acid=50:50:5), Rf= 0.42
¹H-NMR (200 MHz, CDCl₃): δ (ppm): 1.79-1.90 (m, 1H, SH); 2.22-3.11 (m, 7H, S-CH₂-CH-CH₂, NH-CH-CH₂); 4.41-4.53 (m, 1H, NH-CH-COO); 7.11-7.50 (m, 9H, phenyl, phenylene); 6.91-8.12 (m, 3H, furyl); 8.30 (d, 1H, JHH=8.2 Hz, NH);

### N-[3-mercapto-2-(3-pyridylmethyl)propionyl]-4-(2-thiazolyl)-L-phenylalanine stereoisomer A (compound 26)

m.p. 193-196°C
Mass (C.I.) (M+H)⁺= 428
TLC (methylene chloride:methanol:acetic acid=85: 15:1.5), Rf = 0.53
¹H-NMR (200 MHz, DMSO-d₆): δ (ppm): 2.37-3.05 (m, 7H, S-CH₂-CH-CH₂, NH-CH-CH₂); 4.36-4.47 (m, 1H, NH-CH-COO); 7.76-7.90 (m, 2H, thiazolyl); 7.10-8.33 (m, 9H, NH, pyridyl, phenylene);

### N-[3-mercapto-2-(3-pyridylmethyl)propionyl]-4-(2-thiazolyl)-L-phenylalanine stereoisomer B (compound 27)

Mass (C.I.) (M+H)⁺= 428
TLC (methylene chloride:methanol:acetic acid=85:15:1.5), Rf = 0.47
¹H-NMR (200 MHz, DMSO-d₆): δ (ppm): 2.28-3.20 (m, 7H, S-CH₂-CH-CH₂, NH-CH-CH₂); 4.20-4.35 (m, 1H, NH-CH-COO); 7.70-7.90 (m, 2H, thiazolyl); 7.17-8.40 (m, 9H, NH, pyridyl, phenylene);

### N-(2-isobutyl-3-mercapto-propionyl)-4-(2-thiazolyl)-L-phenylalanine (compound 28)

¹H-NMR (200 MHz, DMSO-d₆): δ (ppm): 0.50-1.44 (m, 9H); 1.68-2.25 (m, 4H); 2.79-3.22 (m, 2H); 4.50-4.63 (m, 1H); 7.34-7.85 (m, 4H); 7.73-7.90 (m, 2H); 8.27-8.39 (2d, 1H); Mass (C.I.) (M+H)⁺= 393

### N-[3-mercapto-2-(3-methoxyphenyl)methyl-propionyl]-4-(2-thiazolyl)-L-phenylalanine (compound 29)

Mass (C.I.) (M+H)⁺=457
¹H-NMR (200 MHz, DMSO-d₆+D₂O): δ (ppm): 2.20-3.21 (m, 7H); 3.70 (d, 3H); 4.48 (m, 1H); 6.55-6.86 (m, 3H); 7.00-7.40 (m, 3H); 7.65-7.95 (m, 4H); 8.27-8.45 (bt, CONH);

### N-[3-mercapto-2-(2-fluorophenyl)methyl-propionyl]-4-(2-thiazolyl)-L-phenylalanine (compound 30)

Mass (C.I.) (M+H)⁺=445
¹H-NMR (200 MHz, DMSO-d₆): δ (ppm): 2.20-3.18 (m, 8H); 4.35-4.55 (m, 1H); 6.85-7.35 (m, 6H); 7.65-7.90 (m, 4H); 8.35 (d, 1H);

### N-[3-mercapto-2-(2-thienyl)methyl-proionyl]-4-(2-thiazolyl)-L-phenylalanine (compound 31)

¹H-NMR (200 MHz, DMSO-d₆): δ (ppm): 1.82-3.19 (m, 8H, CH₂-CH₂-CH₂; NH-CH-CH₂); 4.44-4.59 (m, 1H, CH-COO); 6.62-7.91 (m, 9H, aryl); 8.42 (d, 1H, NH);

### N-[3-mercapto-2-(2-furyl)methyl-propionyl]-4-(2-thiazolyl)-L-phenylalanine (compound 32)

¹H-NMR (200 MHz, DMSO-d₆): δ (ppm): 1.79-3.18 (m, 8H, S-CH₂-CH₂-CH₂; NH-CH-CH₂); 4.43-4.58 (m, 4H, CH-NH); 5.79-7.47 (m, 3H, furyl); 7.30-7.85 (m, 4H, phenylene); 7.73-7.91 (m, 2H, thiazolyl); 8.40-8.46 (2d, 1H, NH);

### N-[3-mercapto-2-(3-methyl-5-isoxazolyl)methyl-propionyl]-4-(2-thiazolyl)-L-phenylalanine (compound 33)

¹H-NMR (200 MHz, DMSO-d₆): δ (ppm): 2.00-2.12 (2S, 3H, CH₃-isoxazolyl); 2.28-3.19 (m, 8H, S-CH₂-CH₂-CH₂; CH₂-phenylene); 4.43-4.59 (m, 1H, CH-NH); 5.75-6.03 (2s, 1H, isoxazolyl); 7.29-7.86 (m, 4H, phenylene); 7.74-7.90 (m, 2H, thiazolyl); 8.46-8.53 (2d, 1H, NH);

### Example 8

### In vitro evaluation of the pharmacologic activity

### a) NEP-inhibitory activity

The NEP-inhibitory activity was evaluated in rat kidney cortex membranes prepared according to the procedure described by T. Maeda et al. in Biochim. Biophys. Acta 1983, 731(1), 115-120.

Kidneys were removed from male Sprague-Dawley rats weighing approximately 300 g and kept at 4°C.

Kidney cortex was carefully dissected, finely minced and suspended in a homogenization buffer (10 mM sodium phosphate pH 7.4 containing 1 mM MgCl₂, 30 mM NaCl, 0.02% NaN₃) 1:15 weight/volume.

The tissue was then cold homogenized for 30 seconds using an Ultra-Turrax homogenizer.

Approximately 10 ml of homogenate were layered over 10 ml of sucrose (41% weight/volume) and centrifuged at 31200 rpm for 30 minutes at 4°C in a fixed angle rotor.

The membranes were collected from the buffer/sucrose interface, washed twice with 50 mM TRIS/HCl buffer (pH 7.4) and resuspended into the same buffer for storage in small aliquots at -80°C until use.

The NEP-inhibitory activity was evaluated by using the method described by C. Llorens et al., in Eur. J. Pharmacol., 69, (1981), 113-116, as herinafter reported.

Aliquots of the membrane suspension prepared as above described (concentration 5 µg/ml of proteins) were preincubated in the presence of an aminopeptidase inhibitor (Bestatin - 1 mM) for 10 minutes at 30°C.

[³H][Leu⁵]-enkephaline (15 nM) and buffer TRIS/HCI pH 7.4 (50 mM) were added in order to obtain a final volume of 100 µl.

Incubation (20 minutes at 30°C) was stopped by adding HCI 0.1M (100 µl).

The formation of the metabolite [³H]Tyr-Gly-Gly was quantified by chromatography on polystyrene columns (Porapak Q).

The percentage of inhibition of the metabolite formation in the membrane preparations treated with the compounds of formula I and with the comparative compounds with respect to the untreated membrane preparations was expressed as IC₅₀ (nM) value.

### b) ACE-inhibitory activity

The ACE-inhibitory activity was evaluated according to the method reported in the literature by B. Holmquist et al., in Analytical Biochemistry 95, 540-548 (1979).

50 µM of ACE (250 mU/ml purified by lung rabbit, EC 3.4.15.1 SIGMA) were preincubated with 50 µl of the compound of formula I or of the reference compound or related vehicle in thermostated cuvettes at 37°C.

The reaction was started by adding furylacryloylphenylalanylglycylglycine 0.8 mM (FAPGG-SIGMA).

Contemporaneously, by using a Beckman DU-50 spectrophotometer provided with a program for calculating delta A/minutes and regression coefficients of the enzyme kinetics curves, the absorbance at 340 nm was recorded in continuo for 5 minutes.

The percentage of the enzyme inhibition in the preparations treated with the compounds of formula I or with the comparative compounds with respect to the untreated preparations was expressed as IC₅₀ (nM) value.

The IC₅₀ (nM) values related to the ACE-inhibitory activity and NEP-inhibitory activity of the compounds 16, 18-25, 27-33 and of the comparison compounds Thiorphan and Captopril are reported in the following table 1.

**Table 1**

| ACE-inhibitory and NEP-inhibitory activity, expressed as IC₅₀ (nM) value, of compounds 16, 18-25, 27-33 and of Thiorphan and Captopril. | | |
|---|---|---|
| **Compound** | **ACE-inhibitory activity IC**_{**50**} **(nM)** | **NEP-inhibitory activity IC**_{**50**} **(nM)** |
| 16 | 3.2 | 1.8 |
| 18 | 1.8 | 1.8 |
| 19 | 1.9 | 1.8 |
| 20 | 1.5 | 2.5 |
| 21 | 1.7 | 2.6 |
| 22 | 1.8 | 2.0 |
| 23 | 1.6 | 0.6 |
| 24 | 2.5 | 1.3 |
| 25 | 2.4 | 1 |
| 27 | 9.1 | 17.3 |
| 28 | 5.8 | 1.8 |
| 29 | 4.6 | 9.0 |
| 30 | 10.7 | 11.2 |
| 31 | 8.6 | 1.2 |
| 32 | 8.6 | 4.0 |
| 33 | 7.9 | 4.5 |
| Thiorphan | 99 | 18 |
| Captopril | 4.6 | not active |

The data reported in table 1 show that the compounds of formula I, object of the present invention, are endowed with a significant mixed ACE/NEP inhibitory activity.

Said activity is comparable to that of Captopril, to what it concerns the ACE inhibitory activity, and greater than that of Thiorphan, to what it concerns the NEP inhibitory activity.

### Example 9

### "Ex vivo" evaluation of the pharmacologic activity

### a) NEP-inhibitory activity

The ex vivo NEP-inhibitory activity was evaluated according to the procedure reported in the literature by M. Orlowsky et al., in Biochemistry **1981,** 20, 4942-4950.

The inhibitory activity of the compounds of formula I was evaluated in kidneys of spontaneously hypertensive rats (SHR), 5 minutes after i.v. injection (0.6 and 21 µmoles/kg) and 30 minutes, 60 minutes and 4 hours after oral administration (30 µmoles/kg) of the tested compounds.

After the removal of the kidneys from SHR, the renal tissue was homogenized and incubated for 15 minutes at 37°C in the presence of Glutaryl-Ala-Ala-Phe-2-naphthylamide (GAAP), as a substrate, and aminopeptidase M at pH 7.6.

The reaction was stopped by adding an aqueous solution at 10% of trichloroacetic acid.

The released 2-naphthylamine was determined by adding fast garnet dye (2 ml).

Enzyme reaction rates were determined by measuring the increase in the optical density at 524 nm (OD₅₂₄) with respect to a standard obtained with 2-naphthylamine complexed with fast garnet.

The NEP-inhibitory activity of the tested compounds was expressed as percentage of NEP-inhibition in SHR kidneys.

### b) ACE-inhibitory activity

The ex vivo ACE-inhibitory activity was evaluated by using a radiometric assay method, as reported in the literature by J. W. Ryan et al. in Biochem. J. (1977), 167, 501-504.

The inhibitory activity of the compounds of formula I was evaluated in lungs of spontaneously hypertensive rats (SHR), 5 minutes after i.v. injection (0.6 and 21 µmoles/kg) and 30 minutes, 60 minutes and 4 hours after oral administration (30 µmoles/kg) of the tested compounds.

After the removal of the lungs from SHR, the lung tissue was homogenized and incubated for 2 hours at 37°C in the presence of [³H]Hyp-Gly-Gly, as a substrate.

The reaction was stopped by adding hydrochloric acid.

The released radiolabelled hyppuric acid was extracted with ethyl acetate and counted by liquid scintillation spectrometry, according to conventional methods.

The ACE-inhibitory activity of the tested compounds was expressed as percentage of ACE-inhibition in SHR lungs.

As an example, the percentages of basal enzymatic activity obtained in the ex vivo tests after intravenous or oral administration of compound 16 and of comparison compounds R-1, R-2, R-3 and R-4 are reported in the following table 2.

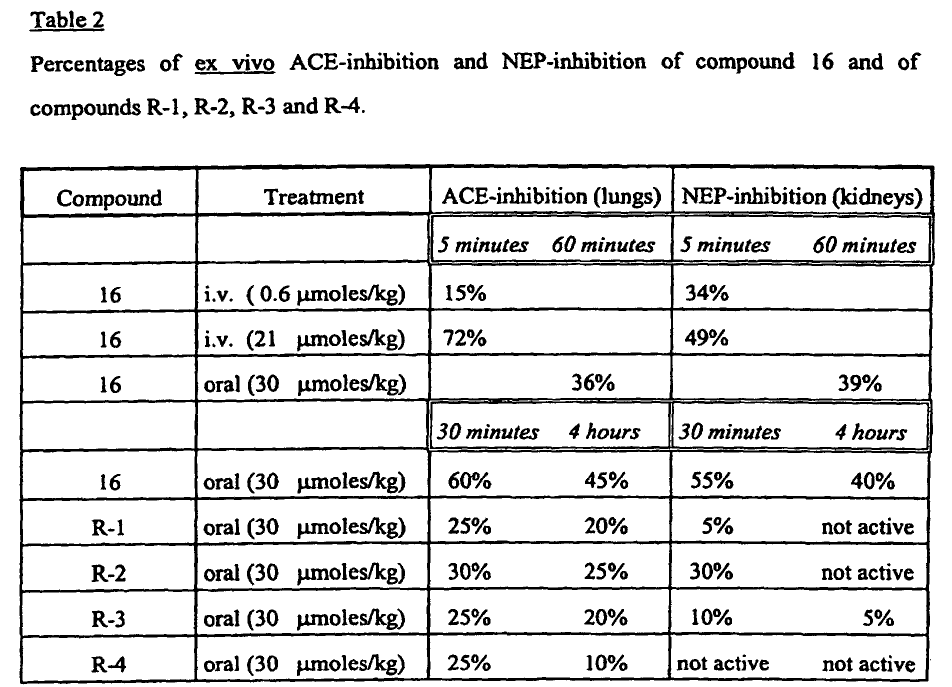

The data reported in table 2 confirm that the compounds of formula I, object of the present invention, are endowed with a significant ACE/NEP-inhibitory activity after intravenous administration as well as after oral administration.

The ex-vivo ACE/NEP-inhibitory activity of the compounds of formula I, moreover, results to be significantly higher than that of the comparison compounds.

## Claims

1. A compound of formula 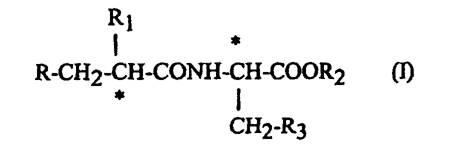 wherein
R is a mercapto group or a R₄COS group convertible in the organism to mercapto group;
R₁ is a straight or branched C₂-C₄ alkyl group or an aryl or arylalkyl group having from 1 to 6 carbon atoms in the alkyl moiety wherein the aryl is a phenyl or a 5 or 6 membered aromatic heterocycle with one or two heteroatoms selected from the group consising of nitrogen, oxygen and sulphur, optionally substituted with one or more substituents, the same or different, selected from the group consisting of halogen atoms, hydroxy groups, alkoxy, alkyl, alkylthio, alkylsulphonyl or alkoxycarbonyl groups having from 1 to 6 carbon atoms in the alkyl moiety, C₁-C₃ alkyl groups containing one or more fluorine atoms, carboxy groups, nitro groups, amino or aminocarbonyl groups, acylamino groups, wherein the acyl group is that of an aliphatic or aromatic carboxylic acid selected from acetic, propionic, butyric and benzoic acid, aminosulphonyl groups, mono- or di-alkylamino or mono- or di-alkylaminocarbonyl groups having from 1 to 6 carbon atoms in the alkyl moiety;
R₂ is a hydrogen atom, a straight or branched C₁-C₄ alkyl or a benzyl group;
R₃ is a phenyl group substituted by a 5 or 6 membered aromatic heterocycle with one or two heteroatoms selected from the group consisting of nitrogen, oxygen and sulphur, being the phenyl and the heterocyclic groups optionally substituted with one or more substituents, the same or different, as indicated for R₁;
R₄ is a straight or branched C₁-C₄ alkyl group or a phenyl group;
the carbon atoms marked with an asterisk are stereogenic centers;
and pharmaceutically acceptable salts thereof.

2. A compound of formula I according to claim 1 wherein R₃ is a phenyl group substituted in position 4 with a heterocyclic group.

3. A compound of formula I according to claim 2 wherein R₁ represents an arylalkyl group optionally substituted with one or more substituents, the same or different, selected from the groups consisting of halogen atoms, hydroxy, alkyl or alkoxy groups.

4. A compound of formula I according to claim 3 wherein R₁ represents a phenylalkyl group optionally substituted with one or more substituents, the same or different, selected among halogen atoms, hydroxy, alkyl or alkoxy groups.

5. A compound of formula I according to claim 1 in the form of a salt with an alkali metal selected from the group consisting of sodium, lithium and potassium.

6. A process for the preparation of a compound of formula I according to claim 1 comprising the reaction between a compound of formula 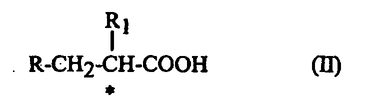 wherein
R and R₁ have the above reported meanings;
and a phenylalanine derivative of formula 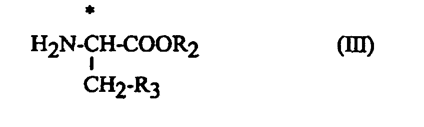 wherein
R₂ and R₃ have the above reported meanings.

7. A pharmaceutical composition containing a therapeutically effective amount of a compound of formula I according to claim 1 in admixture with a carrier for pharmaceutical use.

8. A pharmaceutical composition according to claim 6 for the treatment of cardiovascular diseases.

9. A compound selected from the group consisting of:
N-(3-phenylcarbonylthio-2-phenylmethylpropionyl)-4-(2-thiazolyl)-L-phenylalanine methyl ester;
N-(3-phenylcarbonylthio-2-phenylmethylpropionyl)-4-(2-pyridyl)-L-phenylalanine methyl ester;
N-(3-phenylcarbonylthio-2-phenylmethylpropionyl)-4-(3-pyridyl)-L-phenylalanine benzyl ester;
N-(3-phenylcarbonylthio-2-phenylmethylpropionyl)-4-(2-furyl)-L-phenylalanine methyl ester;
N-(3-phenylcarbonylthio-2-phenylmethylpropionyl)-4-(5-pyrimidinyl)-L-phenylalanine ethyl ester;
N-(3-phenylcarbonylthio-2-phenylmethylpropionyl)-4-(2-pyrazinyl)-L-phenylalanine methyl ester;
N-(3-phenylcarbonylthio-2-phenylmethylpropionyl)-4-(2-thienyl)-L-phenylalanine methyl ester;
N-(3-mercapto-2-phenylmethylpropionyl)-4-(2-thiazolyl)-L-phenylaianine;
N-(3-mercapto-2-phenylmethylpropionyl)-4-(2-pyridyl)-L-phenylalanine;
N-(3-mercapto-2-phenylmethylpropionyl)-4-(3-pyridyl)-L-phenylalanine;
N-(3-mercapto-2-phenylmethylpropionyl)-4-(2-furyl)-L-phenylalanine;
N-(3-mercapto-2-phenylmethylpropionyl)-4-(5-pyrimidinyl)-L-phenylalanine;
N-(3-mercapto-2-phenylmethylpropionyl)-4-(2-pyrazinyl)-L-phenylalanine;
N-(3-mercapto-2-phenylmethylpropionyl)-4-(2-thienyl)-L-phenylalanine;
and the stereoisomers (2S) or (2R) thereof.

10. Use of a compound according to claim 1 in the manufacture of a medicament for the treatment of cardiovascular disease.

## Patentansprüche

1. Verbindung der Formel 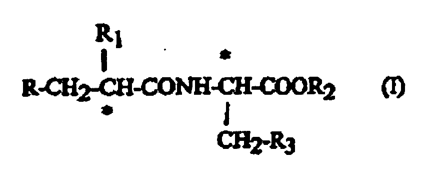 wobei
R eine Mercaptogruppe oder eine R₄COS-Gruppe ist;
R1 eine gerade oder verzweigte C₂-C₄-Alkylgruppe oder eine Aryl oder Alkarylgruppe mit 1 bis 6 Kohlenstoffatomen im Alkylteil ist, wobei das Aryl ein Phenyl oder ein 5- oder 6-gliedriger Heterozyklus ist, mit 1 oder zwei Heteroatomen, die gewählt werden aus der Gruppe von Stickstoff, Sauerstoff und Schwefel, wahlweise mit einem oder mehreren Substituenten substituiert, die gleich oder verschieden sein können, und gewählt werden aus der Gruppe von Halogenatomen, Alkoxy-, Alkyl-, Alkylthio-, Alkylsulphonyl- oder Alkyxycarbonylgruppen, die 1 bis 6 Kohlenstoffatome im Alkylteil haben, C₁-C₃-Alkylgruppen, die ein oder mehrere Fluoratome enthalten, Carboxylgruppen, Nitrogruppen, Amino- oder Aminokarbonylgruppen, Acylaminogruppen, wobei die Acylgruppe gewählt wird unter Essigsäure, Propionsäure, Buttersäure und Benzoesäure, Aminosulphonylgruppen, Mono-oder Dialkylamino oder Mono-oder Dialkylaminocarbonylgruppen mit 1 bis 6 Kohlenstoffatomen im Alkylteil;
R2 Wasserstoff oder eine lineare oder verzweigte C₁-C₄-Alkyl- oder Benzylgruppe ist;
R3 eine Phenylgruppe ist, die substituiert ist mit einem 5-oder 6-gliedrigen aromatischen Heterozyklus mit ein oder zwei Heteroatomen, die gewählt sind aus der Gruppe von Stickstoff, Sauerstoff und Schwefel, wobei die Phenylgruppen und die heterozyklischen Gruppen wahlweise substituiert sein können mit einem oder mehreren, gleichen oder verschiedenen Substituenten wie für R angegeben;
R4 eine lineare oder verzweigte C₁-C₄-Alkyl- oder Phenylgruppe ist;
die mit Sternchen gekennzeichneten Kohlenstoffatome Chiralitätszentren sind;
und deren pharmazeutisch akzeptable Salze.

2. Verbindung der Formel I nach Anspruch 1, wobei R₃ eine Phenylgruppe ist, die in Position 4 mit einer heterozyklischen Gruppe substituiert ist.

3. Verbindung der Formel I nach Anspruch 2, wobei R₁ eine Arylalkylgruppe darstellt, die wahlweise substituiert ist mit einem oder mehreren Substituenten, die gleich oder verschieden sein können, und gewählt werden aus der Gruppe von Halogenatomen, Hydroxy-, Alkyl- oder Alkoxygruppen.

4. Verbindung der Formel I nach Anspruch 3, wobei R₁ eine Phenylalkylgruppe darstellt, die wahlweise substituiert ist mit einem oder mehreren Substituenten, die gleich oder verschieden sein können, und gewählt werden aus der Gruppe von Halogenatomen, Hydroxy-, Alkyl- oder Alkoxygruppen.

5. Verbindung der Formel I nach Anspruch 1, in Form eines Salzes mit einem Alkalimetall, das gewählt wird aus der Gruppe von Natrium, Lithium und Kalium.

6. Verfahren für die Herstellung einer Verbindung der Formel I nach Anspruch 1, umfassend die Reaktion zwischen einer Verbindung der Formel 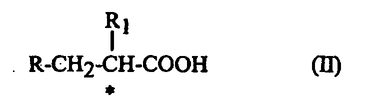 wobei R und R1 die oben angegebenen Bedeutungen haben;
und einem Phenylalaninderivat der Formel 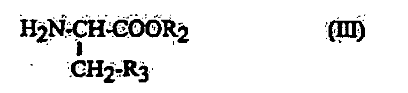 wobei R₂ und R₃ die oben angegebenen Bedeutungen haben.

7. Pharmazeutische Zusammensetzung, die eine therapeutisch wirksame Menge einer Verbindung der Formel I nach Anspruch 1 umfasst, gemischt mit einem Trägermaterial für den pharmazeutischen Gebrauch.

8. Pharmazeutische Zusammensetzung nach Anspruch 6 für die Behandlung von Herz-Kreislauf-Erkrankungen.

9. Verbindung, die gewählt ist aus der Gruppe von:
N-(3-Phenylcarbonylthio-2-phenylmethylpropionyl)-4-(2-thiazolyl)-L-phenylalanin-methylester;
N-(3-Phenylcarbonylthio-2-phenylmethylpropionyl)-4-(2-pyridyl)-L-phenylalanin-methylester;
N-(3-Phenylcarbonylthio-2-phenylmethylpropionyl)-4-(3-pyridyl)-L-phenylalanin-benzylester;
N-(3-Phenylcarbonylthio-2-phenylmethylpropionyl)-4-(2-furyl)-L-phenylalanin-methylester;
N-(3-Phenylcarbonylthio-2-phenylmethylpropionyl)-4-(5-pyrimidyl)-L-phenylalanin-ethylester;
N-(3-phenylcarbonylthio-2-phenylmethylpropionyl)-4-(2-pyrazinyl)-L-phenylalanin-methylester;
N-(3-phenylcarbonylthio-2-phenylmethylpropionyl)-4-{2-thienyl)-L-phenylalanin-methyl-ester;
N-(3-Mercapto-2-phenylmethylpropionyl)-4-(2-thiazonyl)-L-phenylalanin;
N-{3-Mercapto-2-phenylmethylpropionyl)-4-(2-pyridyl)-L-phenylalanin;
N-{3-Mercapto-2-phenylmethylpropionyl)-4-(3-pyridyl)-L-phenylalanin;
N-{2-Mercapto-2-phenylmethylpropionyl)-4-(3-furyl)-Lphenylalanin;
N-(3-Mercpto-2-phenylmethylpropionyl)-4-{5-pyrimidinyl)-L-phenylalanin;
N-{3-Mercapto-2-phenylmethylpropionyl)-4-(2-pyrazinyl)-L-phenylalanin;
N-(3-mercapto-2-phenylmethylpropionyl)-4-(2-thienyl)-L-phenylalanin; und deren (2S) oder (2R) Stereoisomere.

10. Verwendung einer Verbindung nach Anspruch 1 für die Herstellung eines Medikaments zur Behandlung von Herz-Kreislauf-Krankheiten.

## Revendications

1. Composant de formule : 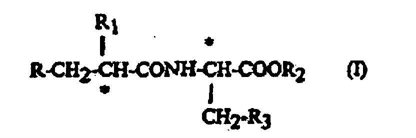 selon laquelle R est un groupe mercapto ou un groupe R₄COS convertible dans l'organisme en un groupe mercapto ;
R₁ est un groupe alkyle C₂-C₄ linéaire ou ramifié ou un aryle ou un groupe arylalkyle ayant de 1 à 6 atomes de carbone dans la fraction alkyle, selon laquelle l'aryle est un phényle ou un hétérocyle aromatique de 5 à 6 membres avec un ou deux hétéroatomes choisis parmis le groupe comprenant l'azote, l'oxygène, et le souffre, éventuellement substitué avec un ou plusieurs substituants, identiques ou différentes, choisis parmis le groupe qui comprend les atomes d'halogène, les groupes hydroxy, les groupes alcoxy, alkyle, alkylthio, alkylsulphonyle, ou alcoxycarbonyle ayant de 1 à 6 atomes de carbone dans la fraction alkyle, les groupes alkyle C₁-C₃ contenant un ou davantage d'atomes de fluor, de groupes carboxy, de groupes nitro, de groupes amino ou aminocarbonyle, de groupes acylamino, selon laquelle le groupe acyle correspond à un acide carboxylique aliphatique ou aromatique choisi parmis l'acide acétique, propionique, butyrique et benzoïque, les groupes aminosulfoniques, les groupe mono- ou di-alkylaminocarbonyle ayant de 1 à 6 atomes de carbone dans la fraction alkyle ;
R₂ est un atome d'hydrogène, un groupe alkyl C₁-C₄ linéaire ou ramifié, ou un groupe benzyle ;
R₃ est un groupe phényle substitué par un hétérocyle aromatique de 5 à 6 membres avec deux hétéroatomes choisis parmis le groupe qui comprend l'azote, l'oxygène et le souffre, les groupes phényle et hétérocyclique étant éventuellement substitués par un ou davantage de substituants, identiques ou différent tels qu'indiqués pour R₁ ;
R₄ est un groupe alkyle C₁-C₄ linéaire ou ramifié, ou un groupe phényle ;
les atomes de carbone marqués d'une astérisque sont des centres stéréogènes ; et les sels correspondants, acceptables sur le plan pharmaceutique .

2. Composant de formule I, selon la revendication 1, **caractérisée en ce que** R₃ est un groupe phényle substitué en position 4 avec un groupe hétérocyclique,

3. Composant de formule I, selon la revendication 2, **caractérisée en ce que** R₁ représente un groupe arylalkyle éventuellement substitué par un ou davantage de substituants, identiques ou différents, choisis parmis le groupe qui comprend des atomes d'halogène, des groupes hydroxy, alkyle ou alcoxy.

4. Composant de formule I, selon la revendication 3, **caractérisée en ce que** R₁ représente un groupe phénylalkyle éventuellement substitué par un ou davantage de substituants, identiques ou différents, choisis parmis les atomes d'halogène, les groupes hydroxy, alkyle ou alcoxy.

5. Composant de formule I, selon la revendication 1, sous forme d'un sel avec un métal alcalin choisi parmis le groupe qui comprend le sodium, le lithium et le potassium.

6. Procédé pour la préparation d'un composant de formule I selon la revendication 1, qui comprend la réaction entre un composant de formule : 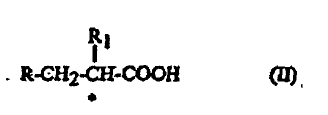 selon laquelle
R et R₁ disposent des significations susmentionnées ;
et un dérivé phénylamine de formule : 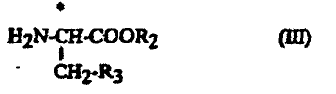 selon laquelle
R₂ et R₃ disposent des significations susmentionnées.

7. Composition pharmaceutique contenant une quantité efficace sur le plan thérapeutique d'un composant de formule I, selon la revendication 1, mélangé à un support d'usage pharmaceutique.

8. Composition pharmaceutique selon la revendication 6 pour le traitement des maladies cardiovasculaires.

9. Composant choisi parmi le groupe comprenant :
l'ester de méthyle N-(3-phénylcarbonylthio-2-phénylméthylpropionyle)-4-(2-thiazolyle)-L-phénylamine ;
l'ester de méthyle N-(3-phénylcarbonylthio-2-phénylméthylpropionyle)-4-(2-pyridyle)-L-phénylamine ; l'ester de benzyle N-(3-phénylcarbonylthio-2-phénylméthylpropionyle)-4-(3-pyridyle)-L-phénylamine ; l'ester de méthyle N-(3-phénylcarbonylthio-2-phénylméthylpropionyle)-4-(2-furyle)-L-phénylamine ;
l'ester d'éthyle N-(3-phénylcarbonylthio-2-phénylméthylpropionyle)-4-(5-pyrimidinyl)-L-phénylamine ;
l'ester d'éthyle N-(3-phénylcarbonylthio-2-phénylméthylpropionyle)-4-(2-pyrazinyle)-L-phénylamine l'ester de méthyle N-(3-phénylcarbonylthio-2-phénylméthylpropionyle)-4-(2-thiényle)-L-phénylamine ; le N-(3-mercapto-2-phénylméthylpropionyle)-4-(2-thiazolyle)-L-phénylamine ;
le N-(3-mercapto-2-phénylméthylpropionyle)-4-(2-pyridyle)-L-phénylamine ;
le N-(3-mercapto-2-phénylméthylpropionyle)-4-(3-pyridyle)-L-phénylamine ;
le N-(3-mercapto-2-phénylméthylpropionyle)-4-(2-furlye)-L-phénylamine ;
le N-(3-mercapto-2-phénylméthylpropionyle)-4-(5-pyrimidinyle)-L-phénylamine ;
le N-(3-mercapto-2-phénylméthylpropionyle)-4-(2-pyrazinyle)-L-phénylamine ;
le N-(3-mercapto-2-phénylméthylpropionyle)-4-(2-thiényle)-L-phénylamine.

10. Utilisation d'un composant selon la revendication 1 pour la fabrication d'un médicament destiné au traitement des maladies cardiovasculaires.
